# FASCICULE DE BREVET EUROPEEN

(11) **EP 1 186 666 B1**
(45) Date de publication et mention de la délivrance du brevet: **12.10.2005**
(21) Numéro de dépôt: 01420162.8
(22) Date de dépôt: 19.07.2001
(51) Int. Cl.: C12N 15/82, A01H 4/00, A01H 5/00

(54) **Utilisation d'inhibiteurs d' hydroxylphenyl pyruvate dioxygenase (HPPD) comme agents de sélection dans la transformation de plantes**
Verwendung von hydroxylphenyl pyruvate dioxygenase ( HPPD ) Inhibitoren als Selektionsmittel für Pflanzentransformation
Use of hydroxylphenyl pyruvate dioxygenase (HPPD) inhibitors as selection agents in plant transformation

(30) Priorité: 11.08.2000 FR 0010601
(43) Date de publication de la demande: 13.03.2002
(73) Titulaire: Bayer CropScience S.A., 69009 Lyon (FR)
(72) Inventeur: Garcon, Frédéric, 69006 Lyon (FR); Pelissier, Bernard, 69370 St Didier au Mont d'Or (FR)
(74) Mandataire: Nowak, Alexander

(56) Documents cités:
- WO-A-96/38567
- WO-A-99/24585
- US-A- 5 217 902

## Description

La présente invention concerne l'utilisation d'inhibiteurs d'hydroxyphyl pyruvate dioxygenase (HPPD, WO 99/24585), comme agents de sélection dans la transformation de cellules végétales et de plantes par génie génétique. La transformation de cellules végétales et de plantes par génie génétique consiste généralement à introduire un gène étranger, ou hétérologue codant pour une protéine d'intérêt, dans le génome des cellules végétales et des plantes qui les contiennent. Ce gène hétérologue une fois intégré dans le génome des cellules végétales est alors exprimé pour conférer aux dites cellules et aux plantes qui les contiennent un nouveau caractère lié à la fonction du gène hétérologue qui sera exprimé.

De nombreuses techniques de transformation des cellules végétales et de plantes par génie génétique ont été développées et abondamment décrites dans la littérature. On pourra distinguer d'une part les méthodes cherchant à introduire un fragment d'ADN porteur du gène hétérologue sous forme dite d'ADN nu. Il s'agit notamment de bombarder des cellules, des protoplastes ou des tissus avec des particules auxquelles sont accrochées les séquences d'ADN. D'autres méthodes peuvent être utilisées telles que la micro-injection ou l'électroporation, ou encore la précipitation directe au moyen de PEG. On distinguera d'autre part les méthodes consistant à utiliser comme moyen de transfert dans la plante un gène chimère hétérologue dans un plasmide Ti d'Agrobacterium tumefaciens ou Ri d'Agrobacterium rhizogenes. L'homme du métier fera le choix de la méthode appropriée en fonction de la nature de la cellule végétale ou de la plante à transformer. On citera notamment les brevets et demandes de brevet suivants: US 4,459,355, US 4,536,475, US 5,464,763, US 5,177,010, US 5,187,073, EP 267,159, EP 604 662, EP 672 752, US 4,945,050, US 5,036,006, US 5,100,792, US 5,371,014, US 5,478,744, US 5,179,022, US 5,565,346, US 5,484,956, US 5,508,468, US 5,538,877, US 5,554,798, US 5,489,520, US 5,510,318, US 5,204,253, US 5,405,765, EP 442 174, EP 486 233, EP 486 234, EP 539 563, EP 674 725, WO 91/02071 et WO 95/06128.

Les méthodes de transformation de cellules végétales comprennent généralement les étapes suivantes:
a) préparation de cellules végétales compétentes aptes à recevoir le gène hétérologue dans un milieu approprié,
b) transformation des cellules compétentes avec le gène hétérologue
c) croissance et sélection des cellules transformées comprenant le gène hétérologue dans un milieu approprié.
   Les cellules végétales compétentes peuvent être des cals embryogéniques, des cultures cellulaires sur support solide ou en suspension, ou des tissus embryogéniques, bien connus de l'homme du métier et largement décrits dans la littérature.
   L'obtention de plantes transgéniques, comprenant le gène hétérologue intégré dans son génome, consiste ensuite à effectuer les étapes suivantes de :
d) régénération de plantes à partir des cellules transformées dans un ou plusieurs milieux appropriés, et le cas échéant,
e) obtention et récupération des graines des plantes transformées fertiles.

La pollinisation des plantes régénérées pour l'obtention des graines des plantes transformées fertiles s'effectue soit par auto-pollinisation, soit par pollinisation croisée avec une variété non transformée de la même plante, ou éventuellement avec une autre variété ayant intégré de manière stable dans son génome un autre gène hétérologue.

Les graines des plantes transformées sont employée ensuite dans des programmes de sélection classique pour l'obtention de nouvelles variétés de plantes transgéniques ayant intégré le gène hétérologue de manière stable dans leur génome. De tels programmes de sélection sont bien connus de l'homme du métier et comprennent l'évaluation des propriétés agronomiques des plantes obtenues et de leur descendance, en particulier vis à vis des propriétés agronomiques liées à l'expression du gène hétérologue.

La sélection des cellules transformées est effectuée au moyen d'un gène marqueur de sélection. De tels gènes marqueurs et leur utilisation pour la transformation d'organismes hôtes sont bien connus de l'homme du métier et largement décrits dans la littérature.

Parmi les gènes codant pour des marqueurs de sélection, on peut d'une part les gènes codant pour des enzymes facilement identifiables comme l'enzyme GUS (ou GFP, " Green Fluorescent Protein "), des gènes codant pour des pigments ou des enzymes régulant la production de pigments dans les cellules transformées. On citera d'autre part les gènes de résistance aux antibiotiques et les gènes de tolérance aux herbicides (bialaphos, glyphosate ou isoxazoles). Dans ce cas, la sélection s'effectue en introduisant dans le milieu approprié pour la croissance et la sélection des cellules transformées un agent de sélection de type antibiotique ou herbicide qui est létal pour les cellules non transformées, seules les cellules comprenant le gène de résistance aux antibiotiques ou aux herbicides étant capables de croître sur le milieu de sélection. De tels gènes marqueurs de sélection sont notamment décrits dans les demandes de brevet EP 242 236, EP 242 246, GB 2 197 653, WO 91/02071, WO 95/06128, WO 96/38567, WO 97/04103 ou WO 99/24585.

Les gènes marqueur de sélection sont introduits dans les cellules hôtes de manière simultanée avec le gène hétérologue, soit dans un même vecteur, les deux gènes étant associés de manière convergente, divergente ou colinéaire (WO 95/06128, US 5 731 179), ou encore dans deux vecteurs employés simultanément pour la transformation des cellules végétales. Dans certaines conditions (US 5 731 179) et notamment dans le cas où le gène hétérologue et le gène marqueur de sélection sont introduit séparément dans deux vecteurs de manière simultanée, le gène hétérologue codant pour une protéine d'intérêt et le gène marqueur de sélection peuvent s'intégrer sur deux chromosomes différents dans le génome de la plante transformée. Il est possible, après récupération de plantes transformées fertiles d'éliminer le gène marqueur pour n'obtenir des plantes transformées ne comprenant que le gène hétérologue codant pour une protéine d'intérêt. Cette élimination se fait par autofécondatrion ou croisement des plantes transformées comprenant le gène hétérologue et le gène marqueur de sélection avec une variété non transformée de la même plante, la ségrégation des deux gènes s'effectuant de manière Mendélienne classique.

Lorsque le gène hétérologue codant pour une protéine d'intérêt est un gène de tolérance herbicide, on peut employer le seul gène hétérologue comme marqueur de sélection dans le procédé de transformation des cellules végétales ou des plantes.

L'utilisation de gènes de tolérance aux herbicides inhibiteurs d'HPPD comme marqueurs de sélection dans les procédés de transformation des cellules végétales et des plantes a été décrite dans la littérature (WO 96/38567, WO 99/24585). L'inhibiteur d'HPPD est introduit dans le milieu de culture des cellules après transformation (étape c), à l'instar des autres agents de sélections selon les pratiques habituelles de l'homme du métier. Les inhibiteurs d'HPPD agissent sur les cellules végétales en inhibant la synthèse des plastoquinones et des caroténoïdes. Cette action conduit à un blanchiment des cellules végétales qui n'est pas dommageable à la croissances des dites cellules, plus particulièrement dans le cas de tissus embryogénique. Seules les cellules végétales transformées comprenant le gène de tolérance aux inhibiteurs d'HPPD restent vertes et peuvent être sélectionnées, se distinguant ainsi des cellules non transformées.

La présente invention consiste en une amélioration d'une telle utilisation de manière à faciliter le processus d'identification et de sélection des cellules transformées. Un second objet de la présente invention consiste à diminuer le temps nécessaire à la sélection des plantes transformées, et à l'obtention de plantes régénérées fertiles. En effet, le processus général de transformation, sélection, régénération et récupération des graines de plantes transformées fertiles peut prendre plusieurs mois selon les plantes considérées, de l'ordre de 10 à 18 mois, notamment pour des plantes telles que le soja. La réduction de cette durée de un ou plusieurs mois constitue un avantage technologique et économique certain.

La présente invention consiste introduire l'inhibiteur d'HPPD dans le milieu de culture des cellules végétales compétentes (étape a) de manière à blanchir les dites cellules avant l'étape de transformation. Les cellules compétentes blanchies sont ensuite transformées avec un gène de tolérance aux inhibiteurs d'HPPD comme marqueur de sélection et les cellules transformées ayant intégré ledit marqueur de sélection dans leur génome verdissent, permettant leur sélection. Un tel procédé permet de réduire le temps nécessaire à la sélection des cellules transformées de plusieurs mois, de l'ordre de 2 à 3 mois.

La présente invention consiste donc en un procédé de transformation de cellules végétales par l'introduction d'un gène hétérologue dans lesdites cellules végétales avec un gène de tolérance aux inhibiteurs d'HPPD comme marqueurs de sélection, ledit procédé comprenant les étapes de
a) préparation et culture de cellules végétales compétentes aptes à recevoir le gène hétérologue dans un milieu approprié,
b) transformation des cellules compétentes avec le gène hétérologue et le marqueur de sélection,
c) croissance et sélection des cellules transformées comprenant le gène hétérologue dans un milieu approprié,
caractérisé en ce que l'on effectue une étape de blanchiment des cellules végétales compétentes avant l'étape de transformation (b), en introduisant dans le milieu approprié de culture des cellules végétales compétentes un quantité appropriée d'inhibiteur d'HPPD.

La présente invention consiste également en l'obtention de plantes transgéniques, comprenant le gène hétérologue intégré dans son génome, consiste ensuite à effectuer les étapes suivantes de :
d) régénération de plantes à partir des cellules transformées sélectionnées dans un ou plusieurs milieux appropriés, et le cas échéant,
e) obtention et récupération des graines des plantes transformées fertiles.

De manière préférentielle les plantes transgéniques obtenues par le procédé selon l'invention sont des plantes transgéniques fertiles.

Les cellules végétales selon l'invention peuvent être des cellules végétales de plantes monocotylédones ou dicotylédones, plus particulièrement des plantes de culture destinées ou non à l'alimentation animale ou humaine, de préférence dicoltylédones, notamment le tabac, le colza, la betterave à sucre, les pommes de terre, le coton, ou le soja, de manière plus préférentielle le soja.

Les cellules végétales compétentes peuvent être des cals embryogéniques, des cultures cellulaires sur support solide ou en suspension, ou des tissus embryogéniques, bien connus de l'homme du métier et largement décrits dans la littérature. De manière avantageuse, les cellules végétales compétentes sont des tissus embryogéniques prolitératifs, maintenus préférentiellement dans un milieu semi solide (In Vitro Cell. Dev. Bioll. Plant 35 :451-455, 1999), et plus particulièrement de cellules de soja. Pour ces cellules compétentes, le blanchiment in vitro lié à l'inhibition de la synthèse des tocophérols n'est pas létale et ne diminue pas la division cellulaire.

La présente invention concerne plus particulier un procédé de préparation d'un soja transgénique comprenant un gène hétérologue intégré dans son génome, ledit procédé comprenant les étapes suivantes :
a1) préparation de tissus embryogéniques prolifératifs par culture d'embryons zygotiques immatures de soja sur un milieu inducteur approprié,
a2) transfert des tissus embryogéniques prolifératifs dans un milieu de culture approprié,
a3) blanchiment des tissus embryogéniques prolifératifs par ajout d'une quantité appropriée d'inhibiteur d'HPPD au milieu de culture,
   b) transformation des tissus embryogénique prolifératifs blanchis par bombardement de particules enrobées de fragments d'ADN comprenant le gène hétérologue et le gène de résistance aux inhibiteurs d'HPPD,
   c) croissance et sélection des cellules transformées comprenant le gène hétérologue et le gène de résistance aux inhibiteurs d'HPPD dans un milieu de culture approprié comprenant une quantité appropriée d'inhibiteur d'HPPD,
   d) régénérations de plantes à partir des cellules transformées séléctionnées dans un ou plusieurs milieux appropriés,
   e) obtention et récupération de graines de soja transformés fertile comprenant le gène hétérologue et le gène de résistance aux inhibiteurs d'HPPD.

De manière avantageuse, les inhibiteurs d'HPPD sont choisis parmi les isoxazoles (EP 418 175, EP 470 856, EP 487 352, EP 527 036, EP 560 482, EP 682 659, US 5 424 276) en particulier l'isoxaflutole, herbicide sélectif du maïs, les dicétonitriles (EP 496 630, EP 496 631), en particulier la 2-cyano-3-cyclopropyl-1-(2-SO2 CH3-4-CF3 phényl) propane-1,3-dione et la 2-cyano-3-cyclopropyl-1-(2-SO2 CH3-4-2,3 C12 phényl) propane-1, 3-dione, les tricétones (EP 625 505, EP 625 508, US 5,506,195), en particulier la sulcotrione ou la mésotrione, ou encore les pyrazolinates. De manière préférentielle, l'inhibiteur d'HPPD est choisi parmi les dikétonitriles, en particulier la 2-cyano-3-cyclopropyl-1-(2-SO2 CH3-4-CF3 phényl) propane-1,3-dione et la 2-cyano-3-cyclopropyl-1-(2-SO2 CH3-4-2,3 Cl2 phényl) propane-1, 3-dione.

La quantité appropriée d'inhibiteur d'HPPD introduite dans le milieu approprié de préparation et de culture des cellules compétentes selon l'invention dépendra d'une part de l'inhibiteur d'HPPD employé et d'autre part des cellules compétents employées, de par leur plante d'origine et leur forme. L'homme du métier saura déterminer cette quantité appropriée par des techniques usuelles de croissance des cellules compétentes à différentes concentrations de l'inhibiteur d'HPPD employé.

De manière préférentielle, la concentration en inhibiteurs d'HPPD est comprise entre 0,5 et 50 mg de matière active par litre de milieu, plus préférentiellement comprise entre 1 et 10 mg/l.

De manière avantageuse, l'inhibiteur d'HPPD est appliqué aux cellules végétales compétentes dans le milieu de culture entre 1 mois et 1 semaines, avant l'étape de transformation, préférentiellement entre 15 et 10 jours. L'homme du métier saura déterminer le moment d'application de l'inhibiteur d'HPPD avant la transformation en fonction des tissus à transformer et de l'inhibiteur d'HPPD et de sa concentration, et de la cinétique de blanchiment des tissus. Il est commun aux techniques de transformation de cellules végétales que les cellules végétales compétentes doivent être repiquées de manière régulière dans des milieux de culture frais. Le temps entre chaque opération de repiquage dépendra en particulier du milieu de culture et de la vitesse de croissance des cellules végétales. Il est généralement de 10 à 15 jours. De manière avantageuse, l'inhibiteur d'HPPD sera introduit dans le milieu de culture frais avant de procéder au repiquage des cellules, généralement au cours du dernier repiquage précédant l'étape de transformation.

Les milieux appropriés pour la préparation et la culture des cellules végétales compétentes comme les milieux appropriés pour la croissance et la sélection des cellules transformées et les milieux pour la régénération des plantes transformées sont des milieux usuels bien connus de l'homme du métier et largement décrits dans la littérature et notamment dans les références citées dans la présente demande de brevet.

Selon un premier mode de réalisation de l'invention, le milieu approprié pour la préparation et la culture des cellules végétales compétentes et le milieu approprié pour la croissance et la sélection des cellules transformées sont identiques et comprennent la même concentration en inhibiteur d'HPPD. Selon un autre mode de réalisation de l'invention, ils ne diffèrent que de par leur concentration en inhibiteur d'HPPD, le premier milieu comprenant une concentration en inhibiteur supérieure au second ou l'inverse. Selon un autre mode de réalisation de l'invention, les milieux diffèrent de par leur composition en éléments nutritifs et hormones nécessaire à la croissance des cellules compétentes avant et après transformation. De manière préférentielle, les deux milieux sont identiques de par leur composition en éléments nutritifs et hormones et leur concentration en inhibiteurs d'HPPD.

Selon un premier mode de réalisation de l'invention, le milieu approprié pour la préparation et la culture des cellule compétentes et/ou le milieu approprié pour la croissance et la sélection des cellules transformées est un milieu D20 décrit Santarém et Finer (In Vitro Cell. Dev. Biol.-Plant 35 : 451-455, 1999), au quel on ajoute une quantité appropriée d'inhibiteur d'HPPD.

Selon un deuxième mode de réalisation de l'invention, le milieu approprié pour la préparation et la culture des cellule compétentes et/ou le milieu approprié pour la croissance et la sélection des cellules transformées est un milieu FNL décrit par Samoylov & coll. (Plant Cell. Rep., 18 :49-54, 1998), dont la composition détaillée est donnée dans les exemples ci-après, auquel on ajoute une quantité appropriée d'inhibiteur d'HPPD. De manière préférentielle, les deux milieux sont des milieux FNL.

Le milieu inducteur approprié est de préférence un milieu D40 tel que défini dans les exemples.

Le milieu de régénération approprié est de préférence un milieu SBP6 décrit par Finer & Nagasawa (Plant Cell. Tissue and Organ Culture 15 : 125-136, 1988) défini dans les exemples ci-après pour la croissance des tissus, puis un milieu tel que décrit par Finner & McMullen (In Vitro Cell. Dev. Biol. 27P :175-182, 1991) pour la conversion des tissus en embryons, puis un milieu MS, en particulier un milieu MS tel que décrit dans les exemples pour la germination des embryons.

Il est entendu dans de qui précède et ce qui suit que lorsque le gène hétérologue que l'on souhaite introduire dans la plante est un gène de tolérance aux inhibiteurs d'HPPD, seul le gène de résistance aux inhibiteurs d'HPPD est introduit dans les cellules végétales.

Selon un mode préférentiel de réalisation de l'invention, l'étape de transformation des cellules compétentes (étape b) est effectuée par la méthode de bombardement de particules, étant entendu que d'autres méthodes équivalentes de transfert d'ADN nu comme l'agitation des cellules compétentes en présence d'ADN et de fibres de silices (Whiskers) peuvent être employées. Le principe de la transformation par bombardement de particules est bien connu de l'homme du métier et largement décrit dans la littérature pour différentes espèces de cellules végétales et de plantes. Pour la transformation des plantes dicotylédones, et du soja en particulier, on citera notamment les références suivantes : Finer & coll. (Plant Cell. Rep. 11 :323-328, 1992). La transformation par bombardement de particules consiste essentiellement à agréger des fragments d'ADN comprenant les gènes à transférer sur des particules métalliques qui sont ensuite bombardées sur les cellules végétales compétentes au moyen de cannons à particules. Les particules comme les appareils permettant le bombardement des cellules compétentes sont bien connus de l'homme du métier, décrits dans la littérature et disponibles dans le commerce. On citera notamment les brevets et demandes de brevet US 4 945 050, EP 270 356, US 5 204 253, EP 434 616, US 5 516 670, EP 535 005 ou US 5 466 587. Selon un mode préférentiel de réalisation de l'invention, les particules métalliques sont des particules fonctionnalisées par le greffage de silicones aminées telles que décrites dans le brevet US 6 068 980.

De manière préférentielle, les gènes de tolérance aux inhibiteurs d'HPPD comprennent dans le sens de la transcription une séquence de régulation promotrice fonctionnelle dans les cellules végétales et les plantes, liée de manière fonctionnelle à une séquence d'ADN codant pour une HPPD liée de manière fonctionnelle à une séquence de régulation terminatrice fonctionnelle dans les cellules végétales et les plantes. Les séquences codant pour des HPPD sont des séquences d'HPPD natives, notamment de plantes, de microorganismes, de champignons ou de mammifères, notamment les séquences décrites dans les demandes de brevet WO 96/38567, US 6 087 563, WO 97/49816 ou WO 99/24585. Il s'agit notamment de séquences codant pour des HPPD de *Pseudomonas fluroescens*, d'*Arabidopsis thaliana*, de carotte, de blé, de *Synecocistys*.. Les séquences codant pour des HPPD sont également des séquences mutées dans leur partie C-terminale telles que décrites dans la demande de brevet WO 99/24585 ou des HPPD chimères telles que décrites dans la demande de brevet WO 99/24586. Selon un mode préférentiel de réalisation de l'invention, la séquence d'ADN codant pour une HPPD est une séquence d'HPPD mutée dans sa partie terminale, plus particulièrement une séquence comprenant la mutation W336 telle que décrite dans la demande de brevet WO 99/24585, plus préférentiellement la séquence d'HPPD de *Pseudomonas fluorescens* comprenant la mutation W336 telle que décrite dans la demande de brevet WO 99/24585.

Selon un mode préférentiel de réalisation de l'invention, le gène de tolérance aux inhibiteurs d'HPPD comprend dans le sens de la transcription une séquence de régulation promotrice sélectionnée parmi le promoteur de la petite sous unité de la RuBisCo de tournesol, décrit dans la demande de brevet WO 99/25842, ou le promoteur d'histone d'*Arabidopsis thaliana* combiné à l'enhancer du virus etch du tabac (TEV) te que décrit dans la demande de brevet WO 99/24585, liée de manière fonctionnelle à une séquence d'ADN codant pour une peptide de transit, de préférence un peptide de transit optimisé, tels que définis ci-après, liée de manière fonctionnelle à une séquence d'ADN codant pour une HPPD telle que définie ci-dessus, de préférence une séquence codant pour une HPPD de *Pseudomonas fluorescens* comprenant la mutation W336, liée de manière fonctionnelle à une séquence de régulation terminatrice, en particuler la séquence terminatrice NOS définie ci-après. Les gènes de tolérance aux inhibiteurs d'HPPD correspondants sont représentés dans les figures en annexe par les cartes de plasmides pCH73 et pCH94, et par leurs séquences nucléotidiques :
pCH73 : SEQ ID NO 1, représentation 3'-5'
   Promoteur: 4541-5257
   Peptide de transit optimisé : 4130-4487
   HPPDW336 : 3045-4119
   NOS : 2749-3000
PCH94 : SEQ ID NO 2
   Promoteur : 34-1272
   TEV enhancer : 1292-1421
   Peptide de transit optimisé : 1428-1793
   HPPDW336 : 1795-2869
   NOS: 2914-3165

De manière préférentielle, les gènes hétérologues codant pour une protéine d'intérêt comprennent dans le sens de la transcription une séquence de régulation promotrice fonctionnelle dans les cellules végétales et les plantes, liée de manière fonctionnelle à une séquence d'ADN codant pour une protéine ou un peptide d'intérêt liée de manière fonctionnelle à une séquence de régulation terminatrice fonctionnelle dans les cellules végétales et les plantes.

Les séquences d'ADN codant pour une protéine ou un peptide d'intérêt sont généralement des séquences codant pour des protéines ou des peptides conférant à la plante transformée de nouvelles propriétés agronomiques, ou d'amélioration de la qualité agronomique de la plante transformée.

Parmi les gènes conférant de nouvelles propriétés agronomiques aux plantes transformées, on peut citer les séquences d'ADN codant pour des protéines conférant une tolérance à certains herbicides, ceux conférant une résistance à certains insectes, ceux conférant une tolérance à certaines maladies, etc. De tels gènes sont notamment décrits dans les demandes de brevet WO 91/02071 et WO 95/06128.

Parmi les séquences d'ADN codant pour des protéines conférant une tolérance à certains herbicides aux cellules végétales et aux plantes transformées., on peut citer le gène Bar conférant une tolérance au bialaphos, le gène codant pour une EPSPS appropriée conférant une résistance aux herbicides ayant l'EPSPS comme cible comme le glyphosate et ses sels (US 4,535,060, US 4,769,061, US 5,094,945, US 4,940,835, US 5,188,642, US 4,971,908, US 5,145,783, US 5,310,667, US 5,312,910, US 5,627,061, US 5,633,435, FR 2 736 926), le gène codant pour la glyphosate oxydoréductase (US 5,463,175), ou encore un gène codant pour une HPPD conférant une tolérance aux herbicides ayant pour cible l'HPPD cités précédemment comme les isoxazoles, notamment l'isoxafutole (FR 95 06800, FR 95 13570), les dicétonitriles (EP 496 630, EP 496 631) ou les tricétones, notamment la sulcotrione ou la mésotrione (EP 625 505, EP 625 508, US 5,506,195).

Parmi les séquences d'ADN codant pour une EPSPS appropriée conférant une résistance aux herbicides ayant l'EPSPS comme cible, on citera plus particulièrement le gène codant pour une EPSPS végétale, en particulier de maïs, présentant deux mutations 102 et 106, décrit dans la demande de brevet FR 2 736 926, dénommé ci-dessous EPSPS double mutant, ou encore le gène codant pour une EPSPS isolée d'Agrobacterium décrit par les séquences ID 2 et ID 3 du brevet US 5,633,435, dénommé ci-dessous CP4.

Dans les cas des séquences d'ADN codant pour EPSPS ou HPPD, et plus particulièrement pour les gènes ci-dessus, la séquence codant pour ces enzymes est avantageusement précédée par une séquence codant pour un peptide de transit, en particulier pour le peptide de transit dit peptide de transit optimisé décrit dans les brevets US 5,510,471 ou US 5,633,448.

Parmi les séquences d'ADN codant pour des protéines d'intérêt conférant de nouvelles propriétés de résistance aux insectes, on citera plus particulièrement les protéines Bt largement décrites dans la littérature et bien connues de l'homme du métier. On citera aussi les protéines extraites de bactéries comme Photorabdus (WO 97/17432 & WO 98/08932).

Parmi les séquences d'ADN codant pour des protéines ou peptides d'intérêt conférant de nouvelles propriétés de résistance aux maladies on citera notamment les chitinases, les glucanases, l'oxalate oxydase, toutes ces protéines et leurs séquences codantes étant largement décrites dans la littérature, ou encore les peptides antibactériens et/ou antifongiques, en particulier les peptides de moins de 100 acides aminés riches en cystéines comme les thionines ou défensines de plantes, et plus particulièrement les peptides lytiques de toutes origines comprenant un ou plusieurs ponts disulfures entre les cystéines et des régions comprenant des acides aminés basiques, notamment les peptides lytiques suivants : l'androctonine (WO 97/30082 et WO 99/09189), la drosomicine (WO 99/02717), la thanatine (WO 99/24594) ou l'héliomicine (WO 99/53053).Selon un mode particulier de réalisation de l'invention, la protéine ou peptide d'intérêt est choisi parmi les peptides éliciteurs fongiques, en particulier les élicitines (Kamoun & al., 1993 ; Panabières & al., 1995).

Parmi les séquences d'ADN codant pour des protéines ou peptides modifiant la constitution des plantes modifiées, on peut citer en particulier les séquences d'ADN codant pour des protéines ou peptides modifiant en particulier la teneur et la qualité de certains acides gras essentiels (EP 666 918) ou encore la teneur et la qualité des protéines, en particuliers dans les feuilles et/ou les graines desdites plantes. On citera en particulier les gènes codant pour des protéines enrichies en acides aminés soufrés (Korit, A.A. & al., Eur. J. Biochem. (1991) 195, 329-334 ; WO 98/20133 ; WO 97/41239 ; WO 95/31554 ; WO 94/20828 ; WO 92/14822). Ces protéines enrichies en acides aminés soufrés auront également pour fonction de piéger et stocker la cystéine et/ou la méthionine excédentaire, permettant d'éviter les problèmes éventuels de toxicité liés à une surproduction de ces acides aminés soufrés en les piégeant. On peut citer également des gènes codant pour des peptides riches en acides aminés soufrés et plus particulièrement en cystéines, les dits peptides ayant également une activité antibactérienne et/ou antifongique. On citera plus particulièrement les défensines de plantes, de même que les peptides lytiques de toute origine, et plus particulièrement les peptides lytiques décrits précédemments. On citera également les protéines SAT décrites dans les demandes de brevet WO 00/36127, WO 00/04167 et WO 00/01833.

Comme séquence de régulation promotrice dans les cellules végétales et les plantes, on peut utiliser toute séquence promotrice d'un gène s'exprimant naturellement dans les plantes en particulier un promoteur s'exprimant notamment dans les feuilles des plantes, comme par exemple des promoteurs dits constitutifs d'origine bactérienne, virale ou végétale ou encore des promoteurs dits lumière dépendants comme celui d'un gène de la petite sous-unité de ribulose- biscarboxylase/oxygénase (RuBisCO) de plante ou tout promoteur convenable connu pouvant être utilisé. Parmi les promoteurs d'origine végétale on citera les promoteurs d'histone tels que décrits dans la demande EP 0 507 698, ou le promoteur d'actine de riz (US 5,641,876). Parmi les promoteurs d'un gène de virus de plante, on citera celui de la mosaïque du choux fleur (CAMV 19S ou 35S), ou le promoteur du circovirus (AU 689 311).

On peut encore utiliser une séquence de régulation promotrice spécifique de régions ou de tissus particuliers des plantes, et plus particulièrement des promoteurs spécifiques des graines ([22] Datla, R.& al., Biotechnology Ann. Rev. (1997) 3, 269-296), notamment les promoteurs de la napine (EP 255 378), de la phaseoline, de la glutenine, de l'héliantinine (WO 92/17580), de l'albumine (WO 98/45460), de l'oélosine (WO 98/45461), de l'ATS1 ou de l'ATS3 (PCT/U598/06978, déposée le 20 octobre 1998, incorporée ici par référence).

On peut également employer un promoteur inductible avantageusement choisi parmi les promoteurs de phénylalanine ammoniac lyase (PAL), d'HMG-CoA reductase (HMG), de chitinases, de glucanases, d'inhibiteurs de proteinase (PI), de gènes de la famille PR1, de la nopaline synthase (nos) ou du gène vspB (US 5 670 349, Tableau 3), le promoteur HMG2 (US 5 670 349), le promoteur de la beta-galactosidase (ABG1) de pomme ou le promoteur de l'amino cyclopropane carboxylate syntase (ACC synthase) de pomme (WO 98/45445).

Selon l'invention, on peut également utiliser, en association avec le promoteur la séquence de régulation promotrice, d'autres séquences de régulation, qui sont situées entre le promoteur et la séquence codante, telles que des activateurs de transcription ("enhancer"), comme par exemple l'activateur de translation du virus de la mosaïque du tabac (VMT) décrit dans la demande WO 87/07644, ou du virus etch du tabac (VET) décrit par Carrington & Freed, ou des introns comme l'intron adhl de maïs ou l'intron 1 d'actine de riz.

Comme séquence de régulation terminatrice ou de polyadénylation, on peut utiliser toute séquence correspondante d'origine bactérienne, comme par exemple le terminateur nos d'Agrobacterium tumefaciens, d'origine virale, comme par exemple le terminateur du CaMV 35S, ou encore d'origine végétale, comme par exemple un terminateur d'histone tel que décrit dans la demande EP 0 633 317.

Les séquences codant pour une HPPD comme les séquences codant pour une protéine ou un peptide d'intérêt peuvent comprendre, liés de manière fonctionnelle en 5' ou en 3' des séquences codant pour des signaux d'adressage dans différents compartiments de la cellule végétale, comme les chloroplastes, les mitochondries, la vacuole. De tels signaux sont décrits dans la littérature et bien connus de l'homme du métier. Les peptide de transit chrolroplastiques peuvent être simples, comme un peptide de transit d'EPSPS (US 5,188,642) ou un peptide de transit de celui de la petite sous-unité de ribulose-biscarboxylase/oxygénase (ssu RuBisCO) d'une plante, éventuellement comprenant quelques acides aminés de la partie N-terminale de la ssu RuBisCO mature (EP 189 707) ou encore un peptide de transit multiple comprenant un premier peptide de transit de plante fusionné à une partie de la séquence N-terminale d'une protéine mature à localisation plastidiale, fusionnée à un deuxième peptide de transit de plante tel que décrit dans le brevet EP 508 909, et plus particulièrement le peptide de transit optimisé comprenant un peptide de transit de la ssu RuBisCO de tournesol fusionné à 22 acides aminés de l'extrémité N-terminale de la ssu RuBisCO de maïs fusionnée au peptide de transit de la ssu RuBisCO de maïs tel que décrit avec sa séquence codante dans le brevet EP 508 909.

Les exemples ci-après permettent d'illustrer l'invention pour la transformation du soja, sans toutefois chercher à en limiter la portée.

Toutes les méthodes ou opérations décrites ci-dessous dans ces exemples sont données à titre d'exemples et correspondent à un choix, effectué parmi les différentes méthodes disponibles pour parvenir au même résultat. La plupart des méthodes d'ingénierie des fragments d'ADN sont décrites dans "Current Protocols in Molecular Biology" Volumes 1 et 2, Ausubel F.M. et al , publiés par Greene Publishing Associates et Wiley -Interscience (1989) ou dans Molecular cloning, T.Maniatis, E.F.Fritsch, J.Sambrook,1982.

Le contenu de toutes les références citées dans la description ci-dessus et dans les exemples ci-après est incorporé au contenu de la présente demande de brevet par référence.

### Exemple 1 : Technologie de transformation du soja

La technologie employée pour la transformation du soja a été décrite par Santarém & Finer: Transformation of soybean (Glycine max (L.) Merrill) using proliferative embryogenic tissue maintened on semi-solid medium. In Vitro Cell. Dev. Biol. Plant 35 : 451-455, 1999. Elle comprend les étapes ci-après.

Des embryons immatures zygotiques (de 3 à 4 mm) sont disséqués de manière aseptisée et placés le côté adaxial sur le dessus dans un milieu comprenant du 2,4D (D40).

D 40 est un milieu Murashige and Skoog décrit dans : Murashige & Skoog: A revised medium for rapid growth and bioassays with tobacco tissue cultures. Physiol. Plant. 15 : 473-497; 1962. (mg/l) : NH4NO3: 1650, H3B03: 6,2; CaCl2, 2H2O: 332,2; CoCl2, 6H2O: 0,025; CuSO4, 5H2O: 0,025; Na2EDTA: 37,26; FeSO4, 7H2O: 27,8; MnSO4, 7H2O: 16,9; Na2MoO4, 2H2O: 0,25; KI: 0,83; KNO3: 1900; KH2PO4: 170; ZnSO4, 7H2O: 8,6 avec de la vitamine B5 Gamborg décrite par : Gamborg, Miller and Ojima: Nutrient requirements of suspension cultures of soybean root cells. Exp. Cell Res. 50 : 151-158, 1968 : (mg/l) : myo-inositol: 100; Nicotinic acid: 1; Pyridoxine-Hcl: 1; Thiamine-Hcl: 10; and 40 mg/l of 2,4-D and 6% sucrose; 0,3% gelrite, pH 7,0.

Après 3 semaines de culture en milieu D40, les cotylédons sont transférés sur un milieu D20 qui comprend essentiellement les même éléments que le milieu D40, à l'exception de la concentration en 2,4D qui est abaissée à 20 mg/l et de la concentration en saccharose qui est abaissée de 60 g/l à 30 g/l, à pH 5,7.

Sur ce milieu, les embryons somatiques commencent à proliférer sous forme de d'agrégats compacts, ou « clumps ». Les « clumps » embryogéniques sont ensuite transférés tous les 15 jours sur un nouveau milieu D 20 pour augmenter la production de tissus. Cinq ou six transferts (environ 3 mois) sur ce milieu sont nécessaires pour optimiser leur compétence à la transformation. L'emploi de tissus embryogéniques à un stade plus précoce conduit à des résultats beaucoup plus faibles.

### Transformation des tissus

Le bombardement de particules est employé pour la transformation des tissus embryogéniques.

Préparation des particules: Des particules de tungstène fonctionnalisées (M17) sont préparées selon le brevet US 6 068 980. Les particules sont fonctionnalisées par greffage de silicones aminées comme élément de vectorisation et lavées dans l'éthanol absolu. 2,5 mg de particules dans l'éthanol sont mélangés avec 3 µg d'ADN. Après précipitation ; les particules sont pipetées et employées pour deux tirs avec un canon à particules de type PIG décrit par Finer, Vain, Lones and McMullen dans : Development of the Particle Inflow Gun for DNA delivery to plant cells. Plant Cell Rep. 11 : 323-328, 1992.

Avant le bombardement, les tissus sont séchés sous vide sous une sous une hotte laminaire pendant 5 à 10 mn, puis placés entre deux écrans de 500 µm puis bombardés deux fois.

Après le bombardement, les tissus sont transférés deux fois sur milieu D20 (2x10 jours) avant de débuter la sélection par l'hygromycine (30 mg/l).

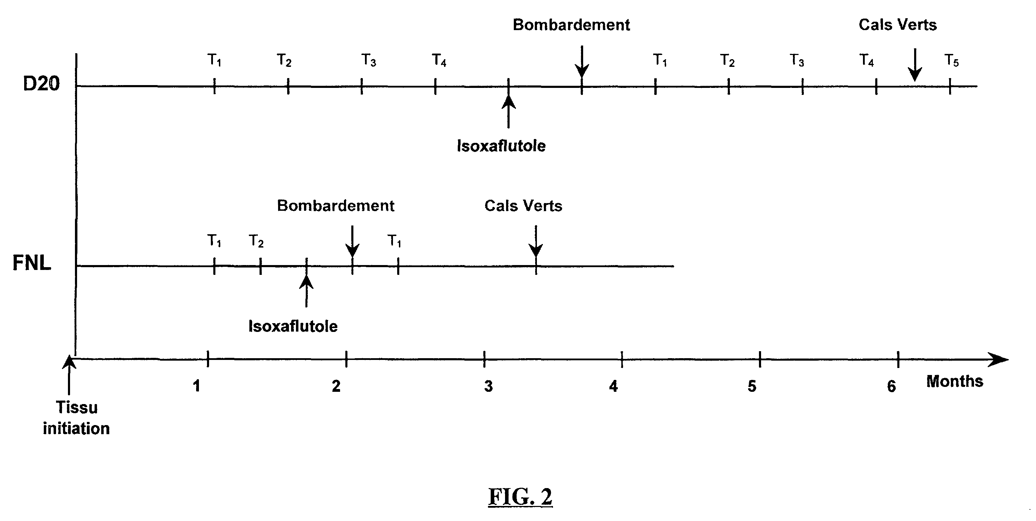

De manière à éviter la manipulation de tissus et de gagner du temps, les cals bombardés sont placés sur un écran de gaze stérile fixé par deux anneaux métalliques (figure 2) qui permettent un contact direct entre les tissus embryogéniques et le milieu solide. Les écrans de gaze sont transférés sur des milieux frais chaque 15 jours jusqu'à ce que des cals verts soient observés. Il est entendu que le principe de transfert de cals décrit ci-dessus n'est pas limité aux cals de soja et à la sélection par l'hygromycine, mais peut être employé pour tout procédé de culture de tissus et de suspensions cellulaires nécessitant un changement fréquent de milieu de culture.

### Amplification et Régénération des tissus

Les cals verts qui poussent sur milieu comprenant de l'hygromycine sont amplifiés pendant 1 mois sur milieu SBP6 décrit par Finer & Nagasawa dans : Development of an embryogenic suspension culture of soybean (Glycine max Merill.) Plant Cell. Tissue and Organ Culture 15 : 125-136, 1988 (mg/l) : Na2EDTA: 37,24; FeSO4, 7H2O: 27,84: MgSO4, 7H2O: 370; MnSO4, H2O: 16,9; ZnSO4, H2O: 8,6; CuSO4, 7H25O: 0,025, CaCl2, 2H2O: 440; KI: 0,83; CoCl2, 6H2O: 0,025; KH2PO4: 170; H3BO3: 6,2; Na2MoO4, 2H2O: 0,25; myo-inositol: 100: nicotinic acid: 1; pyridoxine-HCL: 1; thiamine-HCL: 10; NH4NO3: 800; KNO3: 3000; asparagine: 670; 6% sucrose; 2,4-D: 5, pH 5,7.

Lorsque suffisamment de tissus sont produits, ils sont ensuite convertis en embryons en employant un milieu décrit par Finer & McMullen dans : Transformation of soybean via particle bombardment of embryogenic suspension culture tissue. In Vitro Cell. Dev.Biol. 27P: 175-182, 1991.

Après 3-4 transferts sur ce milieu, les embryons sont séchés à l'air dans une boite de Petri pendant 2 jours avant germination sur un milieu Murashige & Skoog (vitamines B5) à demi force avec 15 g/l de saccharose, 7g/l de phytagar, pH 5,7.

Lorsque les plantes sont bien développées, elles sont transférées dans un substrat à base de tourbe "jiffy pot" pour une période de 10 jours pour acclimatation avant d'être transférées en serre.

Cette technologie employant l'écran de gaze et la sélection par l'hygromycine permet de régénérer 200 cals par personne et par an.

### Example 2 : Transformation des tissus selon l'invention

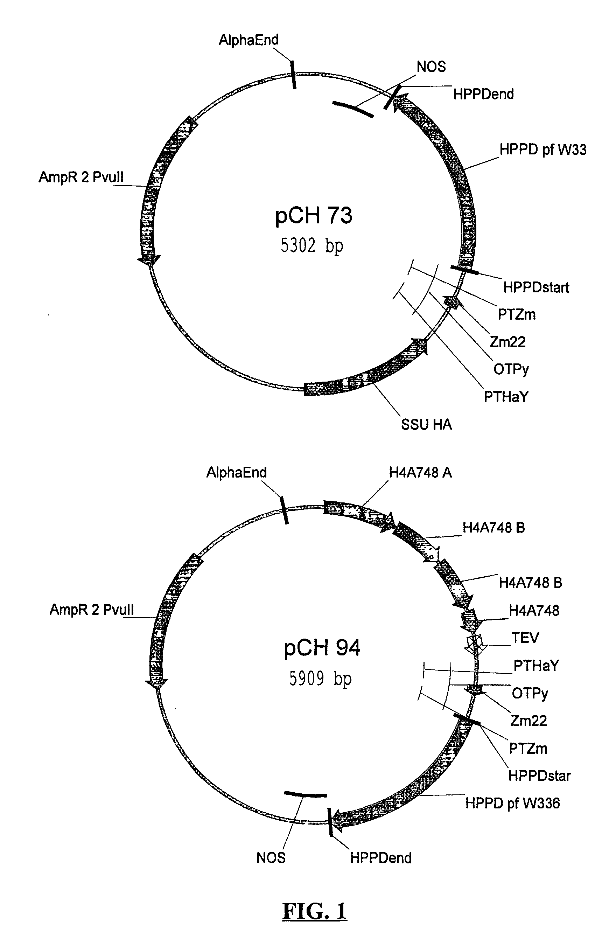

Le même procédé de sélection a été développé pour les inhibiteurs d'HPPD avec un milieu D20 comprenant 2 mg/l d'isoxaflutole ou 0,5 à 5 mg/l de dikétonitriles. Le gène de tolérance aux inhibiteurs d'HPPD employé comme marqueur de sélection est le pCH73 ou le pCH94 représentés sur la figure 1.

10 à 15 jours précédant le bombardement, on introduit dans le milieu D20 l'isoxaflutole ou les dikétonitriles aux concentrations précitées de manière à blanchir les tissus. Après bombardement, les tissus sont placés directement dans le même milieu D20 comprenant 2mg/l d'isoxaflutole ou de dikétonitrile (entre 0,5 to 5 mg/l) et transférés dans un milieu frais tous les 15 jours. Après 4 transferts sur l'isoxaflutole, des cals verts sont identifiés et amplifiés comme décrit dans l'exemple 1. Le temps nécessaire à l'obtention de cals de cellules compétentes pour le bombardement est de 3 mois et demi. La sélection des cellules transformées (cals verts) intervient environ 6 moins suivant l'initiation des cals pour la transformation (figure 2).

Lorsque les tissus sont transférés pour sélection sur l'isoxaflutole, ils sont rapidement blanchis par l'herbicide, sans observer d'inhibition de la croissance. Ceci est dû au fait que l'absence de caroténoïdes, de chlorophylle et de tocophérols n'est pas indispensable à la croissance des tissus.

Les résultats obtenus avec et sans blanchiment préalable sont représentés dans le Tableau 1 ci-après.

**Tableau 1 :**

| **transformation sur milieu D20** | | | | | | |
|---|---|---|---|---|---|---|
| **Blanchiment** | **PCH73** | | | **PCH94** | | |
| **Essai** | 1 | 2 | 3 | 1 | 2 | 3 |
| **Avant Bombardement** | 0 | 0,5 | 0,25 | 0,67 | 1 | 2 |
| **Après Bombardement** | 0 | 0 | 0,25 | 0,67 | 0,33 | 0,25 |

Ils montrent, en particulier pour le pCH94 un plus grand nombre de cals identifiés en amorçant la sélection avant le bombardement selon l'invention. Ce plus grand nombre de cals sélectionnés est lié à l'amélioration du processus de sélection selon l'invention, en facilitant l'identification des cals transformés.

### Exemple 3 : Choix du milieu

De manière à améliorer le processus de sélection selon l'invention, on a cherché à diminuer le nombre de transfert de tissus et le temps nécessaire à l'obtention de cals de cellules compétentes et de cals verts sélectionnés après bombardement. A cet effet, le milieu D20 employé précédemment a été remplacé par un milieu FNL modifié, lequel permet une prolifération rapide des tissus.

Le milieu FNL a été décrit par Samoylov, Tucker, Thibaud-Nissen & Parrott dans : A liquid-medium-based protocol for rapid regeneration from embryogenic soybean cultures: Plant Cell Rep, 18 : 49-54, 1998.

Ce milieu permet d'obtenir plus rapidement des tissus prêts au bombardement, une plus grande embryogénie et des cycles de transfert plus courts.

Composition du milieu FNL (mg/l): Na2EDTA: 37,24; FeSO4, 7H2O: 27,84; MgSO4, 7H2O: 370; MnSO4, H2O: 16,9: ZnSO4, H2O: 8,6: CuSO4, 7H25O: 0,025: CaCl2, 2H2O: 440; KI: 0,83; CoCl2, 6H2O: 0,025; KH2PO4: 170; H3BO3: 6,2; Na2MoO4, 2H2O: 0,25; myo-inositol: 100: nicotinic acid: 1; pyridoxine-HCL: 1; thiamine-HCL: 10; (NH4)2SO4: 460; KNO3: 2820; asparagine: 670; 1% sucrose; 2,4-D: 10; 0,3% gelrite; pH 5,7.

Toutefois, en l'absence de blanchiment préalable, la quantité de tissus à manipuler pendant le processus de sélection reste trop importante, comparable à celle du milieu D20 du fait de la vitesse importante de leur développement.

Le bombardement de tissus blanchis par l'isoxaflutole selon l'invention ne nécessite qu'un seul transfert de cals avant d'obtenir les cals verts sélectionnés, contre 4 en employant le milieu D20.

Le temps nécessaire à l'obtention de cals de cellule compétentes pour le bombardement est de 2 mois, les cals transformés verts étant sélectionnés environ 3 mois suivant l'initiation des cals pour la transformation (figure 2).

Les résultats de transformation avec les plasmides pCH73 et pCH94 sont reportés sur le tableau 2 ci-après.

**Tableau 2 :**

| **Transformation sur milieu FNL** | | | | | | | |
|---|---|---|---|---|---|---|---|
| **Blanchiment** | **pCH73** | | | **PCH94** | | | |
| **Essai** | 1 | 2 | 3 | 1 | 2 | 3 | 4 |
| **Avant Bombardement** | 1 | 1,5 | 0,33 | 1 | 1 | 0,33 | 1,5 |
| **Après Bombardement** | 0,25 | 0 | - | - | - | - | - |

. Le nombre moyen de cals verts sélectionnés par essai de transformation en employant les tissus blanchis selon l'invention est de 1 par tir. Ce tableau montre également qu'avec le blanchiment préalables des tissus, on obtient les même fréquences de transformation pour les deux gènes pCH73 et pCH94, alors que le promoteur ssu RuBisCo de pCH73 est connu pour avoir une expression faibles dans les cals in vitro, contrairement au promoteur d'histone de pCH94.

L'emploi du milieu FNL en combinaison avec le blanchiment préalable des tissus selon l'invention permet de diminuer de manière substantielle le temps nécessaire à la sélection des cals verts et la charge de travail pour tout le processus de transformation des plantes.

## Revendications

1. Procédé de transformation de cellules végétales par l'introduction d'un gène hétérologue dans lesdites cellules végétales avec un gène de tolérance aux inhibiteurs d'hydroxyphenyl pyruvate dioxygenase (HPPD) comme marqueur de sélection, ledit procédé comprenant les étapes de
a) préparation et culture de cellules végétales compétentes aptes à recevoir le gène hétérologue dans un milieu approprié,
b) transformation des cellules compétentes avec le gène hétérologue et le marqueur de sélection,
c) croissance et sélection des cellules transformées comprenant le gène hétérologue dans un milieu approprié,
**caractérisé en ce que** l'on effectue une étape de blanchiment des cellules végétales compétentes avant l'étape de transformation (b), en introduisant dans le milieu approprié de culture des cellules végétales compétentes un quantité appropriée d'inhibiteur d'HPPD.

2. Procédé de préparation de plantes transgéniques, comprenant un gène hétérologue intégré dans son génome, comprenant un procédé de transformation de cellules végétales selon la revendication 1, **caractérisé en ce qu'**il consiste ensuite à effectuer les étapes suivantes de :
d) régénération de plantes à partir des cellules transformées sélectionnées dans un ou plusieurs milieux appropriés, et le cas échéant,
e) obtention et récupération des graines des plantes transformées fertiles.

3. Procédé selon la revendication 2, **caractérisé en ce que** les plantes transgéniques obtenues par le procédé selon l'invention sont des plantes transgéniques fertiles.

4. Procédé selon l'une des revendications 1 à 3, **caractérisé en ce que** les cellules végétales sont choisies parmi les cellules de plantes dicoltylédones, notamment le tabac, le colza, la betterave à sucre, les pommes de terre, le coton, ou le soja.

5. procédé selon la revendication 4, **caractérisé en ce que** les cellules végétales sont des cellules de soja.

6. Procédé selon l'une des revendications 1 à 5, **caractérisé en ce que** les cellules végétales compétentes sont choisies parmi les cals embryogéniques, les cultures cellulaires sur support solide ou en suspension, ou les tissus embryogéniques.

7. Procédé selon la revendication 6, **caractérisé en ce que** les cellules végétales compétentes sont des tissus embryogéniques proliférants.

8. Procédé selon la revendication 7, **caractérisé en ce que** les tissus embryogéniques proliférants sont maintenus dans un milieu semi solide.

9. Procédé selon la revendication 8, **caractérisé en ce que** le milieu semi solide est un milieu FNL.

10. Procédé selon l'une des revendications 1 à 10, **caractérisé en ce que** l'inhibiteur d'HPPD est choisis parmi les isoxazoles, en particulier l'isoxaflutole, les dicétonitriles, en particulier la 2-cyano-3-cyclopropyl-1-(2-SO2 CH3-4-CF3 phényl) propane-1,3-dione et la 2-cyano-3-cyclopropyl-1-(2-SO2 CH3-4-2,3 Cl2 phényl) propane-1, 3-dione, les tricétones, en particulier la sulcotrione ou la mésotrione, ou les pyrazolinates.

11. Procédé selon la revendication 10, **caractérisé en ce que** la concentration en inhibiteurs d'HPPD est comprise entre 0,5 mg/l et 50 mg/l, plus préférentiellement comprise entre 1 mg/l et 10 mg/l

12. Procédé de préparation de plantes transgéniques, comprenant un gène hétérologue intégré dans leur génome, lequel procédé comprend un procédé de transformation de cellules végétales par l'introduction d'un gène hétérologue dans lesdites cellules végétales avec un gène de tolérance aux inhibiteurs d'HPPD comme marqueur de sélection, ledit procédé comprenant les étapes de
a) préparation et culture de cellules végétales compétentes aptes à recevoir le gène hétérologue dans un milieu approprié,
b) transformation des cellules compétentes avec le gène hétérologue et le marqueur de sélection,
c) croissance et sélection des cellules transformées comprenant le gène hétérologue dans un milieu approprié.
d) régénération de plantes à partir des cellules transformées sélectionnées dans un ou plusieurs milieux appropriés, et le cas échéant,
e) obtention et récupération des graines des plantes transformées fertiles, puis
obtention de nouvelles variétés de plantes transgéniques ayant intégré le gène hétérologue de manière stable dans leur génome dans des programmes de sélection classique
**caractérisé en ce que** l'on effectue une étape de blanchiment des cellules végétales compétentes avant l'étape de transformation (b), en introduisant dans le milieu approprié de culture des cellules végétales compétentes un quantité appropriée d'inhibiteur d'HPPD.

13. Procédé selon l'une des revendications 2 à 12, **caractérisé en ce que** le gène marqueur de sélection est éliminé par croisement des plantes transformées comprenant le gène hétérologue et le gène marqueur de sélection avec une variété non transformée de la même plante.

## Patentansprüche

1. verfahren zur Transformation von Pflanzenzellen dadurch, daß man in diese Pflanzenzellen ein heterologes Gen zusammen mit einem Gen für Toleranz gegenüber der Hydroxyphenylpyruvatdioxygenase (HPPD) als Selektionsmarker einbringt, wobei das Verfahren die Schritte
a) Herstellung und Kultivierung von kompetenten Pflanzenzellen, die zum Empfangen des heterologen Gens fähig sind, in einem entsprechenden Medium,
b) Transformation der kompetenten Zellen mit dem heterologen Gen und dem Selektionsmarker,
c) Züchtung und Selektion der transformierten Zellen, die das heterologe Gen enthalten, in einem entsprechenden Medium,
umfaßt, **dadurch gekennzeichnet, daß** man vor dem Transformationsschritt (b) mit den kompetenten Pflanzenzellen einen Bleichschritt durchführt, und zwar dadurch, daß man eine entsprechende Menge eines HPPD-Hemmers in das entsprechende Kulturmedium für die kompetenten Pflanzenzellen einbringt.

2. Verfahren zur Herstellung von transgenen Pflanzen, die ein heterologes Gen in ihr Genom integriert enthalten, das ein Verfahren zur Transformation von Pflanzenzellen nach Anspruch 1 umfaßt, **dadurch gekennzeichnet, daß** es anschließend in der Durchführung der Schritte
d) Regeneration von Pflanzen aus transformierten Zellen, die gegebenenfalls in einem oder mehreren entsprechenden Medien selektiert wurden,
e) Produktion und Gewinnung von Samenkörnern der fruchtbaren transformierten Pflanzen
besteht.

3. Verfahren nach Anspruch 2, **dadurch gekennzeichnet, daß** es sich bei den nach dem erfindungsgemäßen Verfahren gewonnenen transgenen Pflanzen um fruchtbare transgene Pflanzen handelt.

4. Verfahren nach einem der Ansprüche 1 bis 3, **dadurch gekennzeichnet, daß** die Pflanzenzellen aus der Gruppe der Zellen von zweikeimblättrigen Pflanzen, insbesondere Tabak, Raps, Zuckerrübe, Kartoffel, Baumwolle oder Sojabohne stammen.

5. Verfahren nach Anspruch 4, **dadurch gekennzeichnet, daß** es sich bei den Pflanzenzellen um Sojazellen handelt.

6. verfahren nach einem der Ansprüche 1 bis 5, **dadurch gekennzeichnet, daß** die kompetenten Pflanzenzellen aus der Gruppe embryogene Kalli, Zellkulturen auf einem festen Substrat und in Suspension oder embryogene Gewebe stammen.

7. Verfahren nach Anspruch 6, **dadurch gekennzeichnet, daß** es sich bei den kompetenten Pflanzenzellen um proliferierende embryogene Gewebe handelt.

8. Verfahren nach Anspruch 7, **dadurch gekennzeichnet, daß** die proliferierenden embryogenen Gewebe in einem halbfesten Medium erhalten werden.

9. Verfahren nach Anspruch 8, **dadurch gekennzeichnet, daß** es sich bei dem halbfesten Medium um ein FNL-Medium handelt.

10. verfahren nach einem der Ansprüche 1 bis 10, **dadurch gekennzeichnet, daß** der HPPD-Hemmer aus der Reihe Isoxazole, insbesondere Isoxaflutol, Diketonitrile, insbesondere 2-Cyano-3-cyclopropyl-1- (2-SO₂ CH3-4-CF₃₋phenyl)propan-1,3-dion und 2-Cyano-3-cyclopropyl-1-(2-SO₂ CH₃-4-2,3-Cl₂-phenyl)propan-1,3-dion, Triketone, insbesondere Sulcotrion oder Mesotrion, oder Pyrazolinate stammt.

11. Verfahren nach Anspruch 10, **dadurch gekennzeichnet, daß** die HPPD-Hemmerkonzentration zwischen 0,5 mg/l oder 50 mg/l, stärker bevorzugt zwischen 1 mg/l und 10 mg/l, liegt.

12. verfahren zur Herstellung von transgenen Pflanzen, die ein heterologes Gen in ihr Genom integriert enthalten, wobei dieses verfahren ein Verfahren zur Transformation von Pflanzenzellen enthält, dadurch, daß man in diese Pflanzenzellen ein heterologes Gen zusammen mit einem Gen für Toleranz gegenüber HPPD-Hemmern als Selektionsmarker einbringt, wobei das Verfahren die Schritte
a) Herstellung und Kultivierung von kompetenten Pflanzenzellen, die zum Empfangen des heterologen Gens fähig sind, in einem entsprechenden Medium,
b) Transformation der kompetenten Zellen mit dem heterologen Gen und dem Selektionsmarker,
c) Züchtung und Selektion der transformierten Zellen, die das heterologe Gen enthalten, in einem entsprechenden Medium,
d) Regeneration von Pflanzen aus transformierten Zellen, die gegebenenfalls in einem oder mehreren entsprechenden Medien selektiert wurden,
e) Produktion und Gewinnung von Samenkörnern der fruchtbaren transformierten Pflanzen, und
anschließendes Erhalten von neuen Sorten von transgenen Pflanzen, die das heterologe Gen stabil in ihr Genom integriert enthalten, in traditionellen Züchtungsprogrammen umfaßt,
**dadurch gekennzeichnet, daß** man vor dem Transformationsschritt (b) mit den kompetenten Pflanzenzellen einen Bleichschritt durchführt, und zwar dadurch, daß man eine geeignete Menge eines HPPD-Hemmers in das entsprechende Kulturmedium für die kompetenten Pflanzenzellen einbringt.

13. Verfahren nach einem der Ansprüche 2 bis 12, **dadurch gekennzeichnet, daß** das Selektionsmarkergen durch Kreuzen der transformierten Pflanzen, die das heterologe Gen und das Selektionsmarkergen enthalten, mit einer nicht transformierten Sorte der gleichen Pflanze eliminiert wird.

## Claims

1. Method for transforming plant cells by introducing a heterologous gene into said plant cells with a gene for tolerance to hydroxyphenyl pyruvate dioxygenase (HPPD) inhibitors as a selection marker, said method comprising the steps of:
a) preparing and culturing competent plant cells capable of receiving the heterologous gene in a suitable medium,
b) transforming the competent cells with the heterologous gene and the selection marker,
c) growing and selecting the transformed cells comprising the heterologous gene in a suitable medium,
**characterized in that** a step for bleaching the competent plant cells is carried out before the transformation step (b), by introducing a suitable amount of HPPD inhibitor into the suitable culture medium of the competent plant cells.

2. Method for preparing transgenic plants comprising a heterologous gene integrated into their genome, comprising a method for transforming plant cells according to Claim 1, **characterized in that** it consists in carrying out the following steps of:
d) regenerating plants from the transformed cells selected in one or more suitable media and, where appropriate,
e) producing and recovering the seeds of the fertile transformed plants.

3. Method according to Claim 2, **characterized in that** the transgenic plants produced using the method according to the invention are fertile transgenic plants.

4. Method according to one of Claims 1 to 3, **characterized in that** the plant cells are chosen from the cells of dicotyledonous plants, in particular tobacco, rapeseed, sugar beet, potatoes, cotton and soya bean.

5. Method according to Claim 4, **characterized in that** the plant cells are soya bean cells.

6. Method according to one of Claims 1 to 5, **characterized in that** the competent plant cells are chosen from embryogenic calluses, cell cultures on a solid support or in suspension, or embryogenic tissues.

7. Method according to Claim 6, **characterized in that** the competent plant cells are proliferating embryogenic tissues.

8. Method according to Claim 7, **characterized in that** the proliferating embryogenic tissues are maintained in a semi-solid medium.

9. Method according to Claim 8, **characterized in that** the semi-solid medium is an FNL medium.

10. Method according to one of Claims 1 to 10, **characterized in that** the HPPD inhibitor is chosen from isoxazoles, in particular isoxaflutole, diketonitriles, in particular 2-cyano-3-cyclopropyl-1-(2-CH3SO2-4-CF3 phenyl)propan-1,3-dione and 2-cyano-3-cyclopropyl-1-(2-CH3SO2-4-2,3-C12 phenyl)propan-1,3-dione, triketones, in particular sulcotrione or mesotrione, and pyrazolinates.

11. Method according to Claim 10, **characterized in that** the concentration of HPPD inhibitors is between 0.5 mg/l and 50 mg/l, more preferably between 1 mg/l and 10 mg/l.

12. Method for preparing transgenic plants comprising a heterologous gene integrated into their genome, which method comprises a method for transforming plant cells by introducing a heterologous gene into said plant cells with a gene for tolerance to HPPD inhibitors as a selection marker, said method comprising the steps of:
a) preparing and culturing competent plant cells capable of receiving the heterologous gene in a suitable medium,
b) transforming the competent cells with the heterologous gene and the selection marker,
c) growing and selecting the transformed cells comprising the heterologous gene in a suitable medium,
d) regenerating plants from the transformed cells selected in one or more suitable media and, where appropriate,
e) producing and recovering the seeds of the fertile transformed plants, then
producing novel varieties of transgenic plants which have stably integrated the heterologous gene into their genome, in conventional selection programmes,
**characterized in that** a step for bleaching the competent plant cells is carried out before the transformation step (b), by introducing a suitable amount of HPPD inhibitor into the suitable culture medium of the competent plant cells.

13. Method according to one of Claims 2 to 12, **characterized in that** the selection marker gene is eliminated by crossing the transformed plants comprising the heterologous gene and the selection marker gene with a nontransformed variety of the same plant.
